# EUROPEAN PATENT APPLICATION

(11) **EP 4 197 476 A1**
(43) Date of publication of application: **21.06.2023**
(21) Application number: 22158160.6
(22) Date of filing: 23.02.2022
(51) Int. Cl.: A61B 34/20, A61B 34/30, G16H 50/70, G16H 40/63, G16H 20/40

(54) **SYSTEM AND METHOD FOR ESTIMATING AND VISUALIZING TRAJECTORIES OF ROBOTICALLY CONTROLLED INTERVENTIONAL DEVICE**

(30) Priority: 16.12.2021 US 202163290117 P
(71) Applicant: Koninklijke Philips N.V., 5656 AG Eindhoven (NL)
(72) Inventor: FOTOUHI, Javad, Eindhoven (NL); BALICKI, Marcin A, Eindhoven (NL); SINHA, Ayushi, Eindhoven (NL); HAASE, Hans Christian, Eindhoven (NL)
(74) Representative: Philips Intellectual Property & Standards

(57) **Abstract**

A method and system enable estimating and visualizing trajectories of an interventional device guided by a robot and configured for insertion into an anatomical structure. The method includes training a neural network model with regard to predicting trajectories of the interventional device based on training data from previous images and corresponding control inputs; receiving image data from an image showing a current position of the interventional device; receiving untriggered control inputs for controlling the robot to guide future movement of the interventional device; predicting a trajectory of the interventional device by applying the image data and the untriggered control inputs to the trained neural network model; displaying the predicted trajectory of the interventional device overlaid on the image of the anatomical structure; triggering the untriggered control inputs to control the robot to guide movement of the interventional device according to the triggered control inputs when the predicted trajectory is acceptable.

## Description

### BACKGROUND

Robotic systems used for interventional procedures (e.g., endovascular procedures) are operated by a user operating a robotic controller that includes interface devices, such as a joystick controller. The robotic systems control movement of various interventional devices, such as catheters or guidewires. The motion and behavior of the interventional devices in a subject (patient) depend on the input that the user triggers from the robotic controller, as well as the shape, properties and position of the interventional devices relative to the subject's anatomy.

In robot-assisted interventional procedures, steering bendable interventional devices through high curvatures, bifurcations and tortuous anatomical structures (e.g., vessels) may be difficult and complicated, which may result in multiple failed attempts that lead to damage to the anatomical structure (e.g., hemorrhage), increased procedure time, increased exposure of the subject to imaging radiation, and/or the need to change the interventional device. Conventional solutions have no way to visualize or otherwise predict potential trajectories of the interventional device when given different inputs to the robotic system. Consequently, the user may apply suboptimal input, resulting in multiple failed attempts.

Therefore, what is needed is a robotic system that predicts a trajectory of an interventional device during an interventional procedure based on control inputs by the user, and provides a display that shows dynamic lines indicating where the control inputs will position the interventional device before actually moving the interventional device.

### SUMMARY

In a representative embodiment, a system is provided for estimating and visualizing trajectories of an interventional device guided by a robot and configured for insertion into an anatomical structure of a subject. The system includes at least one processor; and a non-transitory memory for storing machine executable instructions that, when executed by the at least one processor, cause the at least one processor to: perform training of a neural network model with regard to predicting trajectories of the interventional device based on training data from previous images of the interventional device in the anatomical structure and corresponding control inputs to the robot for guiding movement of the interventional device as shown in the previous images; receive image data from a current image of the anatomical structure, the current image showing a current position of the interventional device with respect to the anatomical structure; receive untriggered control inputs for controlling the robot to guide future movement of the interventional device from the current position; predict a trajectory of the interventional device in the current image by applying the image data and the untriggered control inputs to the trained neural network model; display the predicted trajectory of the interventional device overlaid on the current image of the anatomical structure for a user to determine whether the predicted trajectory is acceptable; receive a trigger command for triggering the untriggered control inputs to control the robot to guide movement of the interventional device according to the triggered control inputs when the predicted trajectory is determined to be acceptable; and receive new untriggered control inputs for controlling the robot to alternatively guide the future movement of the interventional device from the current position when the predicted trajectory is determined to be not acceptable.

In another representative embodiment, a method is provided for estimating and visualizing trajectories of an interventional device guided by a robot and configured for insertion into an anatomical structure of a subject. The method includes performing training of a neural network model with regard to predicting trajectories of the interventional device based on training data from previous images of the interventional device and corresponding control inputs to the robot for guiding movement of the interventional device as shown in the previous images; receiving image data from at least one image of the anatomical structure, the at least one image showing a current position of the interventional device with respect to the anatomical structure; receiving untriggered control inputs for controlling the robot to guide future movement of the interventional device from the current position; predicting a trajectory of the interventional device in the at least one image by applying the image data and the untriggered control inputs to the trained neural network model; displaying the predicted trajectory of the interventional device overlaid on the at least one image of the anatomical structure; triggering the untriggered control inputs to control the robot to guide movement of the interventional device according to the triggered control inputs when the predicted trajectory is determined to be acceptable; and receiving new untriggered control inputs for controlling the robot to alternatively guide the future movement of the interventional device from the current position when the predicted trajectory is determined to be not acceptable.

In another representative embodiment, a system is provided for determining and visualizing trajectories of an interventional device guided by a robot and configured for insertion into an anatomical structure of a subject. The system includes at least one processor; and a non-transitory memory for storing machine executable instructions that, when executed by the at least one processor, cause the at least one processor to: receive previous image data from a previous image of the anatomical structure showing the interventional device and the anatomical structure; determine previous position data of the interventional device from the previous image data to provide positions and a trajectory of the interventional device in the previous image; receive control inputs for controlling the robot to guide movement of the interventional device from the previous position; compute displacement of the interventional device from the previous position based on the received control inputs applied to the previous position data; translate the computed displacement of the interventional device to image coordinates using previous image calibration; and display a predicted trajectory of the interventional device overlaid on the previous image using the image coordinates.

In another representative embodiment, a system is provided for determining and visualizing trajectories of an interventional device guided by a robot and configured for insertion into an anatomical structure of a subject. The system includes at least one processor; and a non-transitory memory for storing machine executable instructions that, when executed by the at least one processor, cause the at least one processor to: perform training of a neural network model with regard to determining control inputs for controlling the robot to guide the interventional device along a given predicted trajectory; receive current image data from a current image of the anatomical structure showing the interventional device and the anatomical structure; display a predicted trajectory of the interventional device overlaid on the current image, where the predicted trajectory of the interventional device is predicted by applying the current image data and the control inputs to the trained neural network model; receive instructions to adjust the displayed predicted trajectory to a desired position; determine control inputs corresponding to the desired position of the predicted trajectory by applying image coordinates of the predicted trajectory in the desired position to the neural network model; and provide the determined control inputs to a robotic controller for controlling the robot to guide the interventional device along the desired predicted trajectory.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 shows an illustrative interventional device with a predicted trajectory and associated uncertainty margins on a display, according to a representative embodiment.
FIG. 2 is a simplified block diagram of a system for estimating and visualizing trajectories of a robotically controlled interventional device on a display including a graphical user interface (GUI), according to a representative embodiment.
FIG. 3 is a simplified block diagram showing an example implementation of training an encoder-decoder neural network architecture, according to a representative embodiment.
FIG. 4 is a flow diagram showing a method of estimating and visualizing trajectories of a robotically controlled interventional device on a display including a GUI, according to a representative embodiment.
FIG. 5 is a flow diagram of a method of determining and visualizing trajectories of a robotically controlled interventional device on a display including a GUI, according to another representative embodiment.
FIG. 6 is a flow diagram of a method of controlling a robotically controlled interventional device by adjusting visualized trajectories of the interventional device on a display including a GUI, according to another representative embodiment.
FIG. 7 is a simplified block diagram showing an example implementation of training an encoder-decoder neural network architecture, according to a representative embodiment.
FIG. 8 is a simplified block diagram showing another example implementation of training an encoder-decoder neural network architecture, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide a system and method that enable a user (interventionalist) of a robotic system to visualize future (predicted) trajectories of a robotically controlled interventional device based on preliminary (intended) control inputs to the robotic system before the control input is applied or executed. The user is therefore able to adjust the preliminary control inputs based on the predicted trajectories before triggering motion of the interventional device. Predicting the trajectory based on the preliminary inputs reduces the number of failed attempts, lowers the risk of puncture or other damage to tissue, lowers exposure to radiation, and generally improves the usability of the robotic system. A robot controller estimates and visualizes the predicted trajectory of the robotically controlled interventional device before the preliminary input is triggered in an uncalibrated robot-patient setting. The estimates and visualizations are based on planned inputs by the user (without actuating the robot), and the predicted trajectory of the interventional device is overlaid on a recent fluoroscopic image.

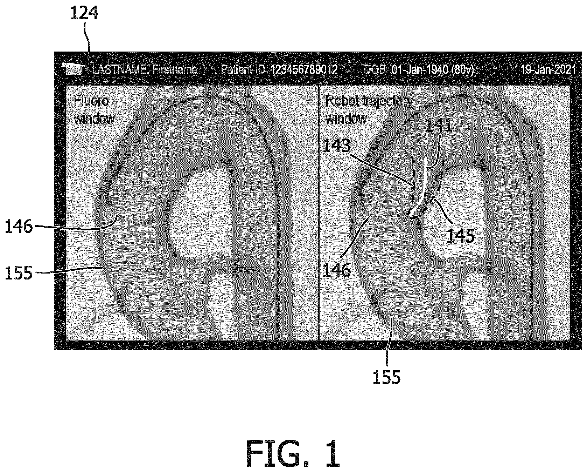

FIG. 1 shows an illustrative interventional device with a predicted trajectory and associated uncertainty margins on a display, according to a representative embodiment.

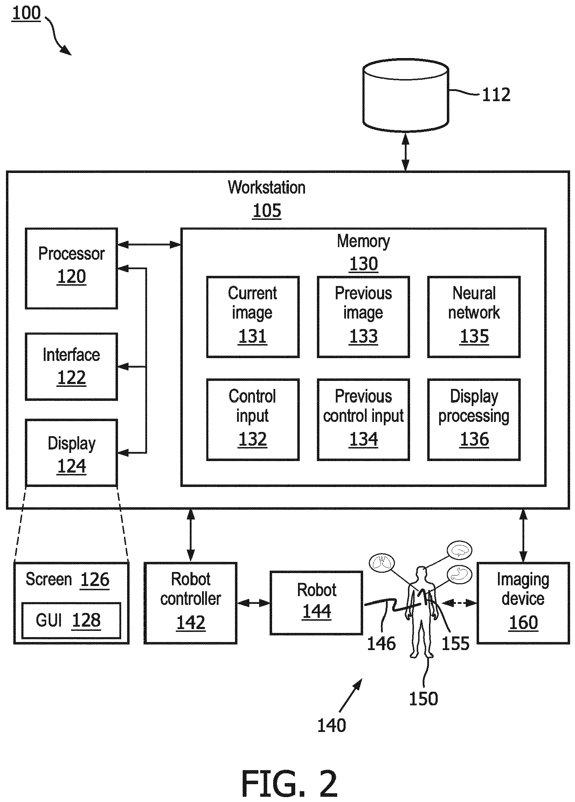

Referring to FIG. 1, a display 124 shows fluoroscopic images of an interventional device 146 configured for insertion into an anatomical structure 155 of a subject, such as a vessel or an artery, for example. The interventional device 146 is guided by a robot controller and a robot, such as robot controller 142 and robot 144 discussed below with reference to FIG. 2. The interventional device 146 may be any compatible medical instrument capable of robotic control, such as a catheter, a guidewire, an endoscope, a camera, a surgical tool, or the like. The image on the left shows the current position of the interventional device 146 within the anatomical structure 155. The image on the right shows a predicted trajectory of the interventional device 146 within the anatomical structure 155 based on control inputs from a user for controlling the robot to guide the interventional device 146 if the control inputs were to be sustained. For example, the control inputs may be untriggered, where the user enters the desired control inputs but does not yet trigger the robot controller 142 to implement the entered control inputs.

In the depicted embodiment, the predicted trajectory is indicated by a centerline 141, and the estimated uncertainty associated with the predicted trajectory is indicated by uncertainty margins indicated by outer-lines 143 and 145 on either side of the centerline 141. The centerline 141 extends longitudinally from a distal end of the interventional device 146 in its current position along an estimated path that the interventional device 146 would follow if the control inputs are sustained (i.e., triggered) by the user. The outer-lines 143 and 145 increasingly depart from the centerline 141 the further the centerline 141 extends away from the current position of the interventional device 146. The outer-lines 143 and 145 thus indicate increasing uncertainty in the prediction the more the predicted trajectory extends from the current position. Alternatively, or in addition, the uncertainty margins may be indicated by color coded pixels defining margins along the centerline of the predicted trajectory, e.g., within the bounds of the outer-lines 143 and 145, where the outer-lines 143 and 145 may or may not be displayed together with the color coded pixels.

The display 124 may be continuously updated as additional images are acquired, and/or as the user changes or fine tunes the control inputs. The predicted trajectory and associated uncertainty may be determined using a neural network module, which has been trained using previously acquired images of the interventional device 146 together with corresponding previously entered control inputs used to position the interventional device 146 as shown in the previous images, as discussed below.

FIG. 2 is a simplified block diagram of a system for estimating and visualizing trajectories of a robotically controlled interventional device on a display including a GUI, according to a representative embodiment.

Referring to FIG. 2, system 100 includes a workstation 105 for implementing and/or managing the processes described herein with regard to controlling movement of an interventional device 146 within an anatomical structure 155 of a subject (patient) 150. The workstation 105 includes one or more processors indicated by processor 120, one or more memories indicated by memory 130, a user interface 122 and a display 124. The processor 120 interfaces with a robotic system 140 through a controller interface (not shown), where the robotic system 140 includes a robot controller 142 and a robot 144. The robot controller 142 is configured to control movement of the robot 144 in response to user input received through the user interface 122 or a separate robotic control interface (not shown). The robot 144 is attached to or integrated with the interventional device 146. The robot 144 may include segments, joints, servo motors and other control features operable for moving and positioning the interventional device 146 in multiple degrees of freedom (DOFs) in response to control signals received from the robot controller 142. In the depicted embodiment, the robot controller 142 is shown separately from processor 120 in the workstation 105 for purposes of illustration. It is understood, though, that the all or part of the functionality of the robot controller 142 may be incorporated into the processor 120, or vice versa, without departing from the scope of the present teachings.

The processor 120 also interfaces with an imaging device 160 through an imaging interface (not shown). The imaging device 160 may be any of various types of medical imaging device/modality, including a fixed or mobile C-arm fluoroscopy system, an X-ray imaging device, a computerized tomography (CT) scan device, a magnetic resonance (MR) imaging device, a positron emission tomography (PET) scan device, or an ultrasound imaging device, for example. The imaging device 160 may include single or multiple imaging modalities.

The memory 130 stores instructions executable by the processor 120. When executed, the instructions cause the processor 120 to implement one or more processes for estimating and visualizing trajectories of robotically controlled interventional device 146 on the display 124. For purposes of illustration, the memory 130 is shown to include software modules, each of which includes the instructions corresponding to an associated capability of the system 100.

The processor 120 is representative of one or more processing devices, and may be implemented by field programmable gate arrays (FPGAs), application specific integrated circuits (ASICs), a digital signal processor (DSP), a general purpose computer, a central processing unit, a graphical processing unit, a computer processor, a microprocessor, a microcontroller, a state machine, programmable logic device, or combinations thereof, using any combination of hardware, software, firmware, hard-wired logic circuits, or combinations thereof. Any processing unit or processor herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 130 may include main memory and/or static memory, where such memories may communicate with each other and the processor 120 via one or more buses. The memory 130 may be implemented by any number, type and combination of random access memory (RAM) and read-only memory (ROM), for example, and may store various types of information, such as software algorithms, artificial intelligence (AI) machine learning models, and computer programs, all of which are executable by the processor 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

The system 100 may also include a database 112 for storing information that may be used by the various software modules of the memory 130. For example, the database 112 may include image data from previously obtained images of the subject 150 and/or of other similarly situated subjects having the same or similar interventional procedures as the subject 150. The stored image data may be used for training an AI machine learning model, such as a neural network model, for example, as discussed below. The database 112 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 112 comprises tangible storage mediums for storing data and executable software instructions and is non-transitory during the time data and software instructions are stored therein. The database 112 may be secure and/or encrypted, or unsecure and/or unencrypted. For purposes of illustration, the database 112 is shown as a separate storage medium, although it is understood that it may be combined with and/or included in the memory 130, without departing from the scope of the present teachings.

The processor 120 may include or have access to an artificial intelligence (AI) engine, which may be implemented as software that provides artificial intelligence (e.g., neutral network models) and applies machine learning described herein. The AI engine may reside in any of various components in addition to or other than the processor 120, such as the memory 130, an external server, and/or the cloud, for example. When the AI engine is implemented in a cloud, such as at a data center, for example, the AI engine may be connected to the processor 120 via the internet using one or more wired and/or wireless connection(s).

The user interface 122 is configured to provide information and data output by the processor 120, the memory 130 and/or the robot controller 142 to the user and/or for receiving information and data input by the user. That is, the user interface 122 enables the user to enter data and to control or manipulate aspects of the processes described herein, and also enables the processor 120 to indicate the effects of the user's control or manipulation. All or a portion of the user interface 122 may be implemented by a graphical user interface (GUI), such as GUI 128 viewable on the display 124, discussed below. The user interface 122 may include one or more interface devices, such as a mouse, a keyboard, a trackball, a joystick, a microphone, a video camera, a touchpad, a touchscreen, voice or gesture recognition captured by a microphone or video camera, for example.

The display 124 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 124 includes a screen 126 for viewing internal images of the subject 150, along with various features described herein to assist the user in accurately and efficiently reading the images, as well as the GUI 128 to enable the user to interact with the displayed images and features. The user is able to personalize the various features of the GUI 128, discussed below, by creating specific alerts and reminders, for example.

Referring to the memory 130, the various modules store sets of data and instructions executable by the processor 120 to estimate and visualize predicted trajectories of the robotically controlled interventional device 146. Current image module 131 is configured to receive and process a current (first) image of the anatomical structure 155 of the subject 150 for display on the display 124. The current image is the image currently being viewed by the user during the interventional procedure. The current image may be received in real time from the imaging device 160 during a contemporaneous current imaging session of the subject 150. Alternatively, the current image may be an image of the subject 150 previously acquired in the current imaging session, where the imaging has been paused, for example, to reduce exposure of the subject 150 to radiation. Likewise, the current image may be retrieved from the database 112, which stores images obtained during previous imaging session(s) or earlier in the current imaging session (from single or multiple imaging modalities). The current image is displayed on the screen 126 to enable analysis by the user and navigation of the robot 144.

Control input module 132 is configured to receive control inputs from the user via the user interface 122 for controlling the robot 144 to guide movement of the interventional device 146. According to various embodiments, the control inputs are initially untriggered, in that the user enters the control inputs without them being executed by the robot controller 142. In this manner, the processor 120 is able to predict the effect of the control inputs on the trajectory of the interventional device 146, as discussed below, prior to the robot controller 142 controlling the robot 144 to actually move the interventional device 146 in response to the control inputs. This enables the user to determine whether the untriggered control inputs are appropriate, e.g., in that their execution would move the interventional device 146 to a desired position by a desired route and would otherwise avoid damaging the anatomical structure 155, before action is taken to implement the control inputs. The control inputs include maneuvering instructions, such as articulation, rotation, translation, distance, velocity, and acceleration of the robot 144, for example, for moving the interventional device 146 in the anatomical structure 155.

Previous image module 133 is configured to receive previous images of the subject 150, for example, from the imaging device 160 and/or the database 112. The previous images include images of the subject 150 acquired earlier in the current imaging session and/or images of the subject 150 acquired during previous imaging sessions involving the same type of interventional procedure. The images may be acquired from a single or multiple imaging modalities. In an embodiment, the previous image module 133 may also receive previous images of other similarly situated subjects having the same or similar interventional procedures as the subject 150 having the same type of interventional procedure. The previous images are used to train a neural network model, discussed below.

Previous control input module 134 is configured to receive previous control inputs associated with the previous images of the previous image module 133. That is, for each previous image, the previous control input module 134 receives the corresponding control inputs provided to the robot controller 142 used to maneuver the interventional device to the position captured by that previous image. The associations between the previous control inputs and the previous images are maintained.

Neural network module 135 is configured to implement a neural network model for predicting trajectories of the interventional device 146 based on preliminary control inputs entered by the user for controlling movements of the robot 144 via the robot controller 142. The neural network model may also estimate the uncertainties associated with the predicted trajectories. For example, the neural network model may determine uncertainty margins (e.g., outer-lines 143 and 145) that are displayed along with a centerline (e.g., centerline 141) on the display 124 to indicate the uncertainty as a function of distance from a current known position of the interventional device 146, as discussed above. In an embodiment, the uncertainty margins may be extended to a volumetric model, for example, a three-dimensional convolutional neural network (CNN). In various embodiments, all or part of the processes provided by the neural network module 135 may be implemented by an AI engine, for example, mentioned above.

The neural network module 135 provides training of the neural network model with regard to predicting trajectories of the interventional device 146 and associated uncertainties based on training data. The training data includes previous images of interventional device(s) and corresponding control inputs for guiding the interventional device(s) as shown in the previous images. For example, the training data may include previous images of the interventional device 146 in the anatomical structure 155 of the subject 150 during the current imaging session and corresponding control inputs to the robot 144 used to guide the interventional device 146 to positions shown in the previous images. The previous images may be retrieved from the previous image module 133 and the corresponding control inputs may be retrieved from the previous control input module 134. Alternatively, the training data may include previous images and corresponding control inputs from previous imaging sessions involving the subject 150, or involving other similarly situated subjects, as discussed above.

Training the neural network model may include capturing spatial context and temporal context of the previous images. The spatial context may be captured using convolutional layers, which consist of a sliding window or kernel representing a matrix of weights that slide over the input images and perform element-wise multiplication with the overlapping part of the input image and summing the results into an output feature map. The temporal context may be captured using temporal connections across layers, such as by using recurrent neural networks (RNNs), long-short term memory (LSTM), transformers, and the like. The neural network training may be performed without supervision (unsupervised training) or with supervision (supervised training), where current image and control input acquisitions may serve as outputs (labels) for previous image and control input acquisitions. Therefore, during the training phase, the neural network model will learn appropriate representations from retrospective data where previous image frames 1, ..., n-1 are used to predict trajectories of the interventional device(s) in the following m time points (image frames n, ..., n+m).

The previous image and previous control input data may be fed together in the earliest layer of the neural network model. The previous image data may include fluoroscopic images, or segmentation maps from different devices or anatomies within the image, for example. The previous control input data may include measurements from the user interface 122 and/or controller console of the robot controller 142, kinematics of the system with regard articulation, rotation and translation, as well as velocity and acceleration. Alternatively, the previous control input data may be used at an intermediate or latent layer of the neural network model, acting as a transformation applied to the representation learned from the previous image frames 1, ..., n-1. In this case, the control input data may also be passed through a series of fully connected layers before merging with the convolutional network. The predicted output in the future image frames n, ..., n+m will produce different trajectories for different robot transformations or control input data. Another implementation may use two separate neural networks, one for the imaging data and one for robot control data. In this case, the two neural networks will share weights or feature maps at some intermediary layers. The training of the neural network model is performed iteratively, where at each iteration, a batch of corresponding previous image and corresponding previous control input data are fed into the neural network model. The training is preceded by minimizing a similarity loss, such as a binary-cross-entropy or intensity loss, as would be apparent to one skilled in the art.

In addition to training the neural network model, the neural network module 135 predicts the trajectory of the interventional device 146 by applying a current image from the current image module 131 and control inputs from the control input module 132 to the trained neural network model. The output of the neural network model provides the predicted trajectory and the uncertainty of the predicted trajectory as a function of the distance the predicted trajectory extends from the current position of the interventional device 146. The distance or time in the future for which the predicted trajectory is generated may be a function of velocity of the interventional device 146 (per degree of freedom or linked to current projection). The gain may be adjusted by the user.

That is, the neural network module 135 receives image data from the current image of the anatomical structure 155, showing a current position of the interventional device 146 with respect to the anatomical structure 155, and receives control inputs for controlling the robot 144 to guide future movement of the interventional device 146 from the current position. The control inputs are untriggered, so that the predicted trajectory may be determined before the robot controller 142 actually implements the control inputs. The control inputs may include current robot parameters and configuration space, such as joystick inputs, force, torque, and forward and inverse kinematics, for example. The neural network module 135 may also input relevant segmentation maps associated with the current image. The neural network model provided by the neural network module 135 predicts the trajectory of the interventional device 146 in the current image, as well as the associated uncertainty, by applying the image data and the untriggered control inputs to the trained neural network model, discussed above.

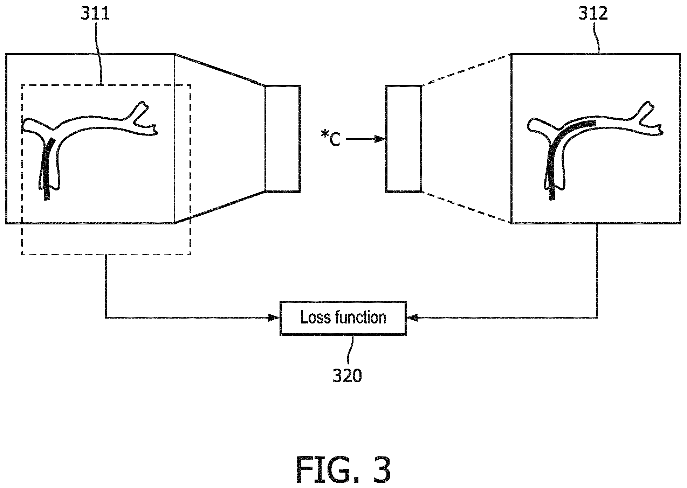

FIG. 3 is a simplified block diagram showing an example implementation of training an encoder-decoder neural network architecture, according to a representative embodiment. The encoder-decoder neural network architecture may be used where the current image is input into the encoder portion of the neural network, which generates a reduced dimension latent representation of the input image. The encoder and decoder portions may contain multiple blocks to capture temporal dependencies, such as RNNs, LSTMs, transformers, and the like. The untriggered control inputs may be applied to the latent representation to produce a transformed latent representation which can be decoded using the decoder portion of the neural network to generate the predicted image containing the predicted trajectory of the interventional device 146.

Referring to FIG. 3, training image 311 may be used as a ground truth image, to which known control inputs C have been applied. During training, the predicted image 312 may be compared to the ground truth image 311, for instance, where the predicted image 312 results from the application of the control inputs C on the current position of the interventional device 146. The comparison may be performed using a loss function 320, as discussed above, and the losses may be used to perform gradient descent to update neural network parameters over multiple iterations in order to minimize the computed loss function over the multiple iterations. By way of example, transforming auto-encoders, which is a similar concept, is described by G. E. Hinton et al. "Transforming auto-encoders" (ICANN 2011), which is hereby incorporated by reference. The predicted trajectory and the uncertainty may also be fed back as input to the neural network module 135 to further train the neural network and assist with the predictions for subsequent untriggered control input data. If the predicted trajectory is determined to be acceptable and the untriggered control inputs are triggered, the resulting trajectory may also be fed back into the neural network as the new current image.

The uncertainty may be estimated using a Monte Carlo dropout algorithm that approximates Bayesian inference for a deep Gaussian process, for example. To trigger dropout, a subset of the neurons is switched off during the forward pass. To compute uncertainty from the dropout, every incoming batch of data is passed through the neural network model multiple times (e.g., ten times). Every time, the dropout algorithm will provide a slightly different form of the neural network model that can subsequently result in another trajectory prediction by the neural network model. The outcomes are aggregated to compute upper and lower bounds for the predicted trajectory. These upper and lower bounds may be visualized on the current image, e.g., as outer-lines 143 and 145, as discussed below, and indicate the uncertainty for the predicted trajectory.

In an embodiment, roadmap images are incorporated into the neural network model. The roadmap images may include registered anatomical structural (e.g., vasculature) information from contrast angiograms and/or pre-procedural imaging. Boundaries of the anatomical structure 155 indicated by the roadmap images affect the expected shape of the interventional device 146. Accordingly, by incorporating the roadmap images, the neural network model may return a different predicted trajectory and uncertainty output, resulting in a different graphical representation of the predicted trajectory.

Display processing module 136 is configured to receive the predicted trajectory and the associated uncertainty, and to display the predicted trajectory and the associated uncertainty on the display 124 overlaid on the current image of the anatomical structure 155. For example, the display processing module 136 may be configured to cause the predicted trajectory to be displayed as a centerline (e.g., centerline 141) and the associated uncertainty to be displayed as uncertainty margins (e.g., outer-lines 143 and 145), as discussed above with reference to FIG. 1. In the event there are multiple coaxial interventional devices 146, each interventional device 146 has its own predicted trajectory display of the corresponding predicted trajectory, where these predicted trajectory displays are inherently coupled to the estimated behavior of both interventional devices 146.

When there is in and out of plane motion of the interventional device 146, the color scheme of predicted trajectory may be correlated with changes in magnitude and direction of the shape of the interventional device 146 along the predicted trajectory. For example, when the interventional device 146 is predicted to move toward a detector of the imaging device 160, the shape of the interventional device 146 is changing little in the current image while the robot 144 is translating the interventional device 146. In this case, the color of predicted trajectory may vary between a first color (e.g., more blue) when the interventional device 146 is expected to shape toward the detector, and a second color (e.g., more green) when the interventional device 146 is expected to shape away from the detector.

The display enables the user to determine whether the predicted trajectory is acceptable with regard to navigating the interventional device 146, taking into account the uncertainty margins. For example, the predicted trajectory may be acceptable when the predicted trajectory advances the interventional device 146 toward a target while avoiding contact with inner walls of the anatomical structure 155, and/or when the predicted trajectory substantially matches a desired trajectory of the interventional device 146. When a volumetric model is used, the display processing module 136 may display a projection of the uncertainty margins or the three-dimensional predicted trajectory overlaid on the current image.

When the predicted trajectory is acceptable, the user triggers the robotic system 140 to move the interventional device 146 in accordance with the control inputs. That is, the robot controller 142 may receive a trigger command, e.g., from the user interface 122, for activating the untriggered control inputs, and in response, control the robot 144 according to the triggered control inputs to guide movement of the interventional device 146. Ideally, the robot 144 moves the interventional device 146 along a path that matches the predicted trajectory, although the path may deviate from the predicted trajectory, particularly when there is relatively high uncertainty. In an embodiment, an actual trajectory of the interventional device 146 may be displayed on the display 124 overlaid on the current image, along with the predicted trajectory, after triggering the untriggered input. Also, the interventional device 146 may be displayed overlaid on the current image while moving the interventional device 146 in accordance with the triggered input. Or, image data from additional (second) images of the anatomical structure 155 may be received from the imaging device 160 while controlling current movement of the interventional device 146 in accordance with the triggered control inputs, in which case the actual trajectory of the interventional device 146 may be overlaid on the additional images while controlling the current movement of the interventional device 146.

Alternatively, the acceptability of the predicted trajectory may be determined automatically, e.g., by the processor 120. For example, the shape of the predicted trajectory may be captured using known segmentation techniques to provide a segmented predicted trajectory, and comparing segmented predicted trajectory to a desired trajectory or to a predetermined set of rules for navigating the interventional device 146. For example, the predicted trajectory may be compared to a centerline or other datum in the anatomical structure 155. When the comparison is within a predetermined margin of error, the processor 120 verifies the predicted trajectory. In another example, the user may draw on the desired trajectory on the GUI 128, and the predicted trajectory may be compared to the desired trajectory to determine the acceptability of the predicted trajectory. The control inputs may then be triggered automatically, or by the user upon notification of the verified trajectory.

When the predicted trajectory is not acceptable, the user may adjust the untriggered control inputs to the robot controller 142 for controlling the robot 144, which would guide the future movement of the interventional device 146 from its current position along an alternative predicted trajectory. In this case, the control input module 132 receives new control inputs from the user via the user interface 122. Again, the new control inputs are initially untriggered, in that the user enters the control inputs without them being executed by the robot controller 142 so that the processor 120 is able to predict the effect of the new control inputs on the trajectory of the interventional device 146 without the robotic system 140 actually moving the interventional device 146.

The neural network module 135 receives the new control inputs, along with a current image of the interventional device 146 in the anatomical structure 155 provided by the current image module 131. The current image may be the same image used to predict the trajectory based on the previous control inputs, or the current image may be a more recently acquired image. The neural network module 135 applies the new control inputs and the current image to the neural network model, which outputs a new (adjusted) predicted trajectory of the interventional device 146 and associated uncertainty. Again, the new predicted trajectory and the uncertainty may be fed back as input to the neural network module 135 to make predictions regarding the future state.

When the new predicted trajectory is acceptable, the user triggers the robotic system 140 to move the interventional device 146 in accordance with the new control inputs. When the new predicted trajectory is not acceptable, process of entering new untriggered control inputs, outputting new predicted trajectories in response, and displaying the new predicted trajectories overlaid on the current image is repeated until an acceptable predicted trajectory is obtained, at which point the corresponding control inputs are triggered to move the interventional device 146 accordingly. This process is essentially continuous in that the new predicted trajectories are displayed substantially immediately following the receipt of the new untriggered control inputs. So, for example, the user may watch the effects that changes to the control inputs have on the displayed predicted trajectories at substantially the same time the changes to the control inputs are being made.

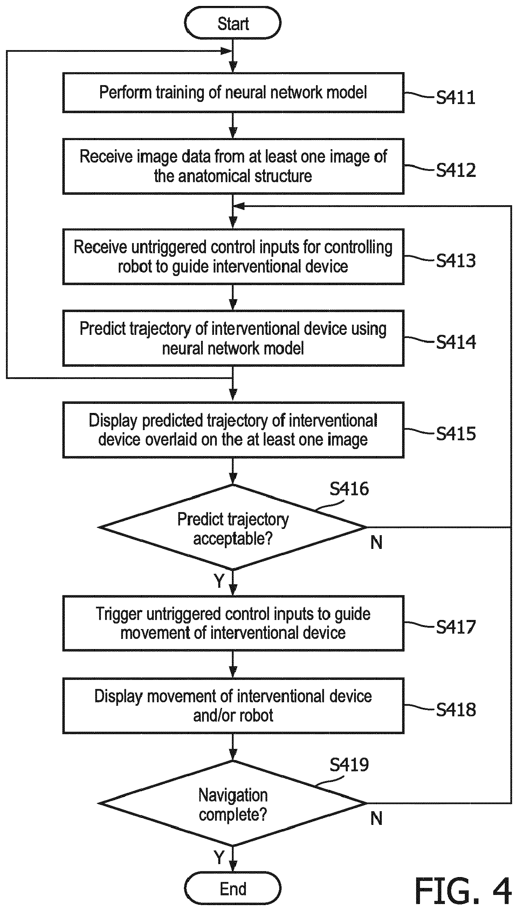

FIG. 4 is a flow diagram of a method of estimating and visualizing trajectories of a robotically controlled interventional device on a display including a GUI, according to a representative embodiment. The method may be implemented by the system 100, discussed above, under control of the processor 120 executing instructions stored as the various software modules in the memory 130, for example.

Referring to FIG. 4, the method includes performing training of a neural network model in block S411 with regard to predicting trajectories of the interventional device and associated uncertainties. The training may be based on training data from previous images of the interventional device and corresponding control inputs to the robot for guiding movement of the interventional device as shown in the previous images.

In block S412, image data is received from at least one image of the anatomical structure, where the at least one image is displayed on a display. The at least one image may be the current image, discussed above. The at least one image shows a current position of the interventional device with respect to the anatomical structure.

In block S413, untriggered control inputs are received for controlling the robot to guide future movement of the interventional device from the current position. The untriggered control inputs may be received via a user interface, resulting from movement of one or more interface devices, such as a mouse, a keyboard, a trackball, a joystick, a microphone, a video camera, a touchpad, a touchscreen, for example. Since the control inputs are untriggered, they have no immediate effect on movement of the robot or the interventional device controlled by the robot.

In block S414, a trajectory of the interventional device in the at least one image is predicted by applying the received image data and the untriggered control inputs to the trained neural network model. Along with the predicted trajectory, the trained neural network model also estimates uncertainty associated with the predicted trajectory. Generally, the uncertainty increases the further the predicted trajectory extends from the current position of the interventional device. Notably, the predicted trajectory and the associated uncertainty are returned to block S411, along with the corresponding untriggered control inputs, to further train the neural network model.

In block S415, the predicted trajectory of the interventional device is displayed on the display overlaid on the at least one image of the anatomical structure. In an embodiment, the estimated uncertainty associated with the predicted trajectory may also be displayed over laid on the at least one image. For example, the predicted trajectory may be shown as a centerline on the at least one image, and the associated uncertainty may be shown as outer-lines on either side of the centerline, as discussed above.

In block S416, it is determined whether the predicted trajectory has been identified as being an acceptable trajectory for purposes of continuing to navigate the interventional device in the anatomical structure. The determination may be made in response to input entered by the user via the user interface accepting or rejecting the predicted trajectory. Alternatively, the acceptability of the predicted trajectory may be determined automatically using known segmentation techniques to provide a segmented predicted trajectory, and comparing segmented predicted trajectory to a desired trajectory or to a predetermined set of rules for navigating the interventional device. For example, the predicted trajectory may be acceptable when it advances the interventional device toward a target while avoiding contact with inner walls of the anatomical structure, and/or when the predicted trajectory substantially matches a desired trajectory of the interventional device.

When the predicted trajectory is not acceptable (S416: No), the process returns to block S413, where new untriggered control inputs are received for controlling the robot. The new untriggered control inputs would guide future movement of the interventional device from the current position differently than the previous untriggered control inputs. Blocks S414 through S416 are then repeated in an attempt to identify an acceptable predicted trajectory.

When the predicted trajectory is acceptable (S416: Yes), the untriggered control inputs are triggered in block S417 to control the robot to guide movement of the interventional device according to the triggered control inputs. In an embodiment, block S417 may be effectively combined with block S416 where the predicted trajectory is determined to be acceptable in response to receiving a command triggering the untriggered control inputs.

Movement of the interventional device and/or the robot in response to the triggered control inputs may be displayed in block S418. The movement of the interventional device may be monitored using the display, enabling continued navigation of the interventional device through the anatomical structure. Actual movement of the interventional device may be displayed by acquiring additional images of the interventional device in real time during the interventional procedure. Alternatively, the movement of the interventional device may be simulated using the same image as a reference image, and applying the triggered control inputs to a navigation model for navigating the interventional device, where the navigation model is essentially the same as the neural network model that was used for the prediction. In an embodiment, since the navigation model is essentially the same as the neural network model used for the prediction, the predicted trajectory may simply be overlaid on the reference image. This overlay may be rendered in a different color or shade, for example, to be distinguished from untriggered predictions for the subsequent frames. Also, when uncertainty associated with the predicted trajectory becomes too high (e.g., exceeds a predetermined threshold), an alert may be provided to the user.

In block S419, it is determined whether navigation of the interventional device in the anatomical structure continues. This determination may be made, for example, when untriggered control inputs continue to be received, e.g., via the user interface, or when a predetermined target in the displayed image has not yet been reached. When it is determined that navigation is continuing (block S419: Yes), the process returns to block S413, where additional untriggered control inputs are received for controlling the robot for guiding future movement of the interventional device. Blocks S414 through S419 are then repeated to form a control loop for interactively guiding the interventional device using the robot. When it is determined that navigation is continuing (block S419: Yes), the process returns. When it is determined that navigation is not continuing (block S419: No), the process ends.

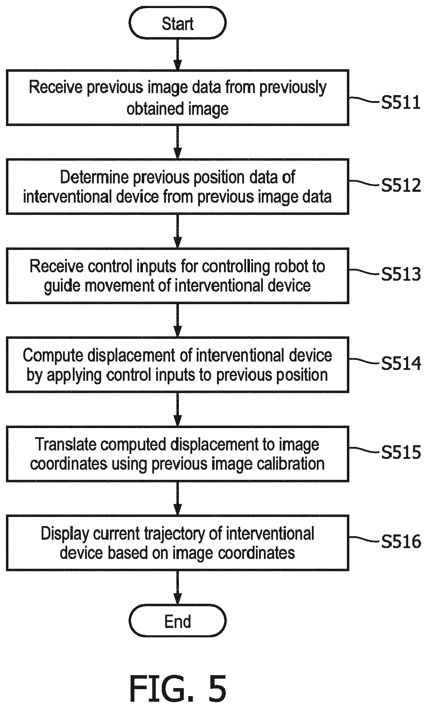

FIG. 5 is a flow diagram of a method of determining and visualizing trajectories of a robotically controlled interventional device on a display including a GUI, according to another representative embodiment. The method may be implemented by the system 100, discussed above, under control of the processor 120 executing instructions stored in the memory 130, for example. In the embodiment of FIG. 5, the trajectory of an interventional device may be estimated without use of a neural network model, thereby avoiding training and application of the neural network.

Referring to FIG. 5, the method includes receiving previous image data from a previously obtained image (e.g., from previous image module 133) of the anatomical structure in block S511. The previous image shows the interventional device and the anatomical structure in the subject at an earlier time. For example, the previous image may show the interventional device at time t=0, while the interventional device is currently at a time t=n, where n>0.

In block S512, previous position data of the interventional device is determined from the previous image data. The previous position data provides a previous position and a trajectory of the interventional device in the previous image. The previous position data may be determined by applying known segmentation techniques to the previous image, for example. The trajectory of the interventional device in particular may be determined by comparing the previous position of the interventional device in the previous image to earlier images of the interventional device. Here, the previous position describes how the interventional device is positioned in the context of the previous image, providing spatial context for the interventional device. The previous trajectory describes how the device moves across multiple previous images, providing temporal context for the interventional device.

In block S513, control inputs are received via the user interface (e.g., from control input module 132) for controlling the robot to guide movement of the interventional device from the previous position. The control inputs may be triggered control inputs resulting in actual movement of the interventional device.

In block S514, displacement of the interventional device is computed by applying the received control inputs to the previous position data from the previous image. The displacement is from the position of the interventional device in the previous position provided by the previous position data to a displaced position in accordance with the control inputs.

In block S515, the computed displacement of the interventional device is translated to image coordinates using previous image calibration of the previous image. For example, in a case where the interventional device may be tracked using some technology that does not involve segmenting the interventional device in image space to provide position and/or trajectory information, tracking data may be in a different coordinate space compared to the image data, thus requiring calibration. However, the calibration information may be used in subsequent images.

In block S516, a current trajectory of the interventional device is displayed based on the image coordinates of the displacement. The current trajectory is displayed overlaid on the displayed previous image. In this manner, movement of the interventional device along the current trajectory is shown on the previous image, so that additional imaging is not necessary during the interventional procedure.

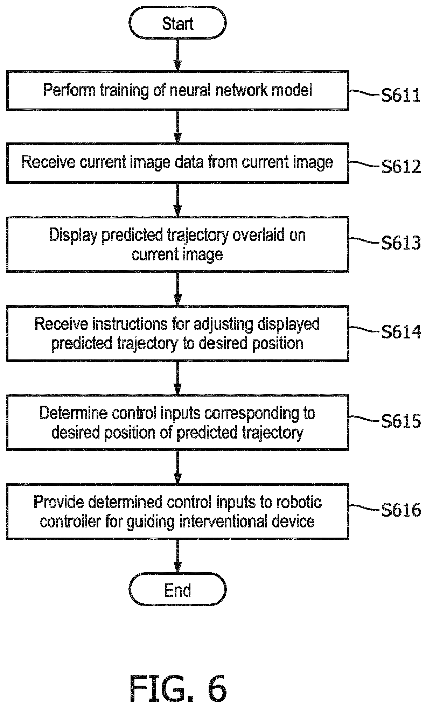

FIG. 6 is a flow diagram of a method of controlling a robotically controlled interventional device by adjusting visualized trajectories of the interventional device on a display including a GUI, according to another representative embodiment. The method may be implemented by the system 100, discussed above, under control of the processor 120 executing instructions stored in the memory 130, for example. The embodiment shown in FIG. 6 is essentially the reverse of the embodiment shown in FIG. 4, where the robot is controlled automatically by the robotic controller in response to instructions by the user that adjust displayed trajectories on the display.

Referring to FIG. 6, the method includes performing training of a neural network model in block S611 with regard to determining control inputs for controlling the robot to guide the interventional device along a given predicted trajectory. The training may be based on training data from previous images of the interventional device and corresponding control inputs to the robot for guiding movement of the interventional device as shown in the previous images.

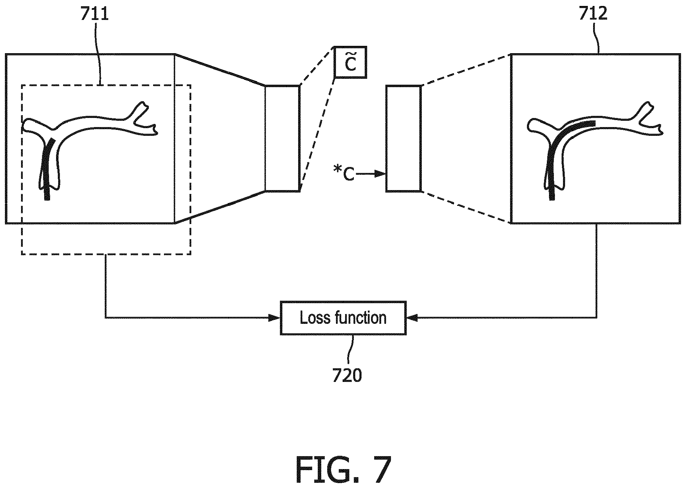

FIG. 7 is a simplified block diagram showing an example implementation of training an encoder-decoder neural network architecture, according to a representative embodiment. The encoder-decoder neural network architecture may be similar to the neural network described above with reference to FIG. 3, where the current image is input into an encoder portion of the neural network, which generates a reduced dimension latent representation of the input image. The latent representation is additionally used to estimate untriggered control inputs which can then be applied to the latent representation to produce a transformed latent representation. The transformed latent representation may be decoded to generate a predicted image containing the predicted trajectory of the interventional device as before.

Referring to FIG. 7, training image 711 may be used as a ground truth image, to which estimated control inputs *C̃* have been applied. During training of the neural network, the predicted image 712 may be compared to training image 711, for instance, the predicted image 712 resulting from application of the estimated control inputs *C̃* on the current position of the interventional device. The comparison may be performed using a loss function 720, as discussed above, and the losses are used to perform gradient descent to update neural network parameters over multiple iterations in order to minimize the computed loss functions. When the corresponding control inputs are known, then the estimated control inputs *C̃* may also be compared with the known or ground truth corresponding control inputs. Both comparisons may be performed using the loss function 720, as discussed above, and a combination of the losses (e.g., average or weighted average) are used to perform gradient descent to update neural network parameters over multiple iterations in order to minimize the computed loss function.

During inference, when future image or control inputs are not known, the desired future trajectory of the interventional device may be specified by the user by drawing the desired trajectory on the GUI, for example. The estimated control inputs *C̃* may be fined-tuned during inference, as well by computing the transformed latent representation and decoding it to generate the predicted image containing the predicted trajectory of the interventional device and comparing it to the user specified desired future trajectory of the device. Comparisons may be performed using loss functions as described above, and the losses may be used to perform gradient descent as described above.

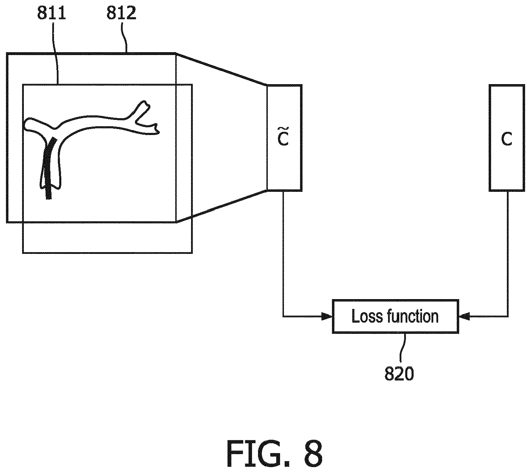

FIG. 8 is a simplified block diagram showing another example implementation of training an encoder-decoder neural network architecture, according to a representative embodiment. Referring to FIG. 8, the current image 812 and the image 811 containing the desired future trajectory of the interventional device (e.g., as indicated by the user drawing on the GUI) may both be input into a neural network (e.g., as separate channels) that directly estimates the untriggered control inputs that would transform the interventional device in the current image to the desired future trajectory. In this implementation, no fine-tuning is required during inference using a trained network. During training of the neural network, the estimated control inputs *C̃* are compared with known or ground truth corresponding control inputs C. Comparison and gradient descent are performed as specified above.

Referring again to FIG. 6, in block S612, current image data is received from a current image (e.g., from current image module 131) of the anatomical structure. The current image shows the anatomical structure in the subject, e.g., obtained by the imaging device 160, and the current position of the interventional device within the anatomical structure.

In block S613, a predicted trajectory of the interventional device is displayed on overlaid on the current image. The predicted trajectory of the interventional device is predicted by applying the current image data and the current control inputs to the trained neural network model, as discussed above. Along with the predicted trajectory, the trained neural network model may also provide estimated uncertainty associated with the predicted trajectory, which may also be displayed.

In block S614, instructions are received from the user to adjust the displayed predicted trajectory to a desired position. For example, the GUI may include a touchscreen that enables the user to drag the predicted trajectory to the desired position on the display screen. Or, as another example, the user may use a mouse to click and drag the predicted trajectory to the desired position.

In block S615, control inputs corresponding to the desired position of the predicted trajectory are determined by applying image coordinates of the predicted trajectory in the desired position to the neural network model, as described above.

In block S616, the determined control inputs are provided to the robotic controller, which automatically controls the robot to guide the interventional device along the desired predicted trajectory in response to the determined control inputs.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although estimating and visualizing trajectories of a robotically controlled interventional device on a display has been described with reference to exemplary embodiments, it is understood that the words that have been used are words of description and illustration, rather than words of limitation. Changes may be made within the purview of the appended claims, as presently stated and as amended, without departing from the scope and spirit of the embodiments. Although estimating and visualizing trajectories of a robotically controlled interventional device on a display has been described with reference to particular means, materials and embodiments, it is not intended to be limited to the particulars disclosed; rather the estimating and visualizing trajectories of a robotically controlled interventional device on a display extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for estimating and visualizing trajectories of an interventional device guided by a robot and configured for insertion into an anatomical structure of a subject, the system comprising:
at least one processor; and
a non-transitory memory for storing machine executable instructions that, when executed by the at least one processor, cause the at least one processor to:
perform training of a neural network model with regard to predicting trajectories of the interventional device based on training data from previous images of the interventional device in the anatomical structure and corresponding control inputs to the robot for guiding movement of the interventional device as shown in the previous images;
receive image data from a current image of the anatomical structure, the current image showing a current position of the interventional device with respect to the anatomical structure;
receive untriggered control inputs for controlling the robot to guide future movement of the interventional device from the current position;
predict a trajectory of the interventional device in the current image by applying the image data and the untriggered control inputs to the trained neural network model;
display the predicted trajectory of the interventional device overlaid on the current image of the anatomical structure for a user to determine whether the predicted trajectory is acceptable;
receive a trigger command for triggering the untriggered control inputs to control the robot to guide movement of the interventional device according to the triggered control inputs when the predicted trajectory is determined to be acceptable; and
receive new untriggered control inputs for controlling the robot to alternatively guide the future movement of the interventional device from the current position when the predicted trajectory is determined to be not acceptable.

2. The system of claim 1, wherein the executed instructions further cause the at least one processor to:
predict an alternative trajectory of the interventional device in the current image by applying the image data and the new untriggered control inputs to the trained neural network model;
display the alternative predicted trajectory of the interventional device overlaid on the current image of the anatomical structure for the user to determine whether the alternative predicted trajectory is acceptable; and
receive the trigger command for triggering the new untriggered control inputs to control the robot to guide movement of the interventional device according to the triggered control inputs.

3. The system of claim 1, wherein the predicted trajectory is acceptable when the predicted trajectory substantially matches a desired trajectory of the interventional device.

4. The system of claim 1, further comprising:
an imaging system configured to acquire the current image of the anatomical structure, and to provide the image data from the current image to the at least one processor; and
a robot controller configured to enable control of the robot in accordance with the triggered control inputs.

5. The system of claim 1, wherein the executed instructions further cause the at least one processor to estimate an uncertainty of the predicted trajectory using the neural network model, and to display the estimated uncertainty with the predicted trajectory overlaid on the current image of the anatomical structure.

6. The system of claim 5, wherein the executed instructions further cause the at least one processor to display the predicted trajectory as a centerline, and to display the estimated uncertainty as outer-lines defining margins along the centerline of the predicted trajectory.

7. The system of claim 5, wherein the executed instructions further cause the at least one processor to display the predicted trajectory as a centerline, and to display the estimated uncertainty as color coded pixels defining margins along the centerline of the predicted trajectory.

8. The system of claim 5, wherein performing the training of the neural network model comprises applying a dropout algorithm for estimating the uncertainty of the predicted trajectory.

9. The system of claim 1, wherein the executed instructions further cause the at least one processor to display an actual trajectory of the interventional device overlaid on the current image, along with the predicted trajectory, after triggering the untriggered control inputs.

10. The system of claim 1, wherein the executed instructions further cause the at least one processor to:
receive additional image data from additional images of the anatomical structure while controlling current movement of the interventional device; and
display an actual trajectory of the interventional device overlaid on the additional images while controlling the current movement of the interventional device.

11. The system of claim 1, wherein performing the training of the neural network model with regard to predicting the trajectories comprises unsupervised training.

12. A method of estimating and visualizing trajectories of an interventional device guided by a robot and configured for insertion into an anatomical structure of a subject, the method comprising:
performing training of a neural network model with regard to predicting trajectories of the interventional device based on training data from previous images of the interventional device and corresponding control inputs to the robot for guiding movement of the interventional device as shown in the previous images;
receiving image data from at least one image of the anatomical structure, the at least one image showing a current position of the interventional device with respect to the anatomical structure;
receiving untriggered control inputs for controlling the robot to guide future movement of the interventional device from the current position;
predicting a trajectory of the interventional device in the at least one image by applying the image data and the untriggered control inputs to the trained neural network model;
displaying the predicted trajectory of the interventional device overlaid on the at least one image of the anatomical structure;
triggering the untriggered control inputs to control the robot to guide movement of the interventional device according to the triggered control inputs when the predicted trajectory is determined to be acceptable; and
receiving new untriggered control inputs for controlling the robot to alternatively guide the future movement of the interventional device from the current position when the predicted trajectory is determined to be not acceptable.

13. The method of claim 12, further comprising:
predicting an alternative trajectory of the interventional device in the at least one image by applying the image data and the new untriggered control inputs to the trained neural network model;
displaying the alternative predicted trajectory of the interventional device overlaid on the at least one image of the anatomical structure; and
triggering the new untriggered control inputs to control the robot to guide movement of the interventional device according to the triggered control inputs.

14. The method of claim 12, wherein the predicted trajectory is displayed as a centerline, and estimated uncertainty of the predicted trajectory is displayed as outer-lines defining margins along the centerline of the predicted trajectory.

15. A computer program product comprising instructions which, when executed on a processor, cause the processor to carry out the method steps of claim 12.
